Europäisches Patentamt

**(19)** European Patent Office

Office européen des brevets

**(11)** Publication number : **0 480 690 A1**

**(12)** EUROPEAN PATENT APPLICATION

**(21)** Application number : 91309243.3

**(22)** Date of filing : 09.10.91

**(51)** Int. Cl.⁵ : **A61K 9/107,** A61K 9/06, A61K 31/415

**(30)** Priority : **10.10.90 US 595337**

**(43)** Date of publication of application :
**15.04.92 Bulletin 92/16**

**(84)** Designated Contracting States :
**CH ES FR GB LI**

**(71)** Applicant : **IOLAB CORPORATION**
**500 Iolab Drive**
**Claremont, California 91711-4881 (US)**

**(72)** Inventor : **Evitts, David Paul**
**8440-C Via Mallorca**
**La Jolla, CA 92037 (US)**
Inventor : **Olejnik, Orest**
**26931 Aldeano Drive**
**Mission Viejo, CA 92691 (US)**
Inventor : **Musson, Donald George**
**2073 Redgrove Way**
**Upland, CA 91786 (US)**
Inventor : **Bidgood, Alison Margaret**
**7349 Springmill Place**
**Rancho Cucamonga, CA 9130 (US)**

**(74)** Representative : **Fisher, Adrian John et al**
**CARPMAELS & RANSFORD 43 Bloomsbury Square**
**London WC1A 2RA (GB)**

**(54)** Aqueous ophthalmic microemulsions of tepoxalin.

**(57)** The invention provides an ophthalmic composition suitable for topical application to the eye comprising an oil in water microemulsion wherein the microemulsion contains tepoxalin in an anti-inflammatory effective concentration.

EP 0 480 690 A1

The invention relates to aqueous ophthalmic microemulsions of tepoxalin.

## Background Of The Invention

Tepoxalin, whose formal chemical name is 3-[5-(4-chlorophenyl)-1-(4-methoxyphenyl)-3-pyrazolyl]-N-hydroxy-N-methylpropanamide, is a nonsteroidal anti-inflammatory drug. It is a potent inhibitor of the cyclooxygenase and lipoxygenase pathways of the arachidonic acid metabolism when administered topically or parenterally. The drug is presently being developed as an ophthalmic topically administered anti-inflammatory composition. Tepoxalin is nearly insoluble in water. Many attempts have been made with various surfactants, cyclodextrins, etc., to enhance its aqueous solubility in order to achieve a homogenous solution rather than a suspension. These attempts have been largely unsuccessful. Tepoxalin, however, is much more soluble in oils than in water and a procedure to disperse the oil in an aqueous phase using surfactants has been discovered and forms the basis of this invention.

A dispersion of oil in water (o/w) can be defined as either a macroemulsion or a microemulsion. A macroemulsion is a cloudy turbid composition with an oil-droplet size of 0.5 to 100 μm. Macroemulsions are usually unstable. A microemulsion is a translucent to transparent composition having a droplet size of 0.005 to 0.5 μm. Microemulsions are usually stable (Stig E. Friberg and Pierre Bothorel, Microemulsions:1 Structure and Dynamics, CRC press, Inc., Boca Raton, Florida, 1987, page 154). The components to generate an emulsion include water, an organic solvent, a surfactant, and possibly a co-surfactant. The o/w system is titrated with the surfactant(s) to a hydrophilic-lipophilic balance (HLB) to obtain a "one-phase transparent o/w dispersion" (H.L. Rosano, J.L. Cavallo and G.B. Lyons, Chapter 16, Microemulsion Systems, vol. 24, ed. H.L. Rosano and M. Clause, Marcel Dekker, Inc., New York and Basel, 1987 page 271). Thermodynamically stable microemulsions can form upon mixing with the proper composition of water, oil, and surfactants. Other emulsions require a high input of energy by sonication, by homogenization, or by shear. (D.M. Lidgate, R.C. Fu and J.S. Fleitman, BioPharm, October, 1989, page 28).

The potential advantages of an emulsion formulation versus aqueous are enhanced solubility for hydrophobic drugs (A. El-Sayed and A. Repta, Int. J. Pharm., 13 (1903) 303), enhanced stability for hydrolytic drugs [P. Grover, Ph.D. Thesis, The University of Connecticut (1984)], and sustained-release characteristics of a drug out of the oil phase [S. Davis, Pharm. Technol., 71 (May 1981); P. Madan, Pharm. Manuf., 51 (June 1985)]. For the above reasons, many investigators have examined the pharmaceutical use of emulsions, as is illustrated by the following citations:

(a) ophthalmic flurbiprofen preparation, Mizushima, Y., Okamoto, H., Sugio, S., Yokoyama, K., Suyama, T., Tohmo, M., Ohumura, M., Konishi, Y., Ichikawa, K. (Kahen Pharmaceutical Co., LTD.), European patent Application, #87304334.3;

(b) dexamethasone acetate, O. Yoichi, S. Takashi, Y. Eiichiro (Shiesido Co., Ltd.) Jpn. Kokai Tokkyo Koho JP 63 10,717 (88 10,717) (CL A61k9/10), 18 Jan 1988, JP Appl. 86/50,219, 07 March 1986; pp. 17;

(c) indomethacin, M. Yu, M. Kawachi, H. Nakajima (Shiesido Co., Ltd.) Jpn. Kokai Tokkyo Koho JP 63,126,542 (88,126,542) (Cl. B01j13/00), 30 May 1988, Appl. 86/273,672, 17 Nov 1986, page 8; and

(d) tolnaftate (transdermal), Y. Ota, E. Yagi, M. Fukuda, T. Suzuki (Shiesido Co., Ltd.) Jpn. Kokai Tokkyo Koho JP 86, 291,518 A2, JP 61,291,518 22 December 86.

The solubility of tepoxalin was tested in several representative oils such as sesame oil, sunflower seed oil, and castor oil. Initial toxicity studies with neat castor oil indicated irritation to the eye (NZW rabbit). This provided an impetus to develop aqueous stable emulsions containing tepoxalin in the hope that they would not be irritating. Acute animal toxicity tests with the microemulsions made in accordance with this invention showed no eye irritation.

## Brief Summary of the Invention

The invention provides an ophthalmic composition suitable for topical application to the eye comprising an oil in water microemulsion wherein the microemulsion contains tepoxalin in an anti-inflammatory effective concentration. The tepoxalin is contained in the oil phase, the oil/surfactant/water interface, and the water phase.

## Detailed Description of the Invention

The pharmacolocically active component of the ophthalmic compositions of the invention is tepoxalin, or 3-[5-(4-chlorophenyl)-1-(4-methoxyphenyl)-3-pyrazolyl]-N-hydroxy-N-methylpropanamide. Tepoxalin and its preparation are described in Wachter et al., U.S. Patent No. 4,826,868, the disclosure of which is incorporated herein by reference.

The oils that can be used in the microemulsions of the invention include castor oil (USP grade), sesame oil, sunflower seed oil, mineral oil, and other non-volatile oils. The preferred class of oils that are used in the invention are the fixed oils (i.e., non-volatile oils of vegetable origin). Castor oil is preferred because of tepoxalin's greater solubility therein, and because previous pharmaceutical uses of castor oil have demonstrated its safety and efficacy. In selecting the oil to be used, it is not necessary that the oil per se dissolve tepoxalin, because some oils in which tepoxalin is not soluble to any great degree will form an aqueous microemulsion with a surfactant such that the aqueous emulsion will dissolve sufficient tepoxalin for the microemulsion to be useful. Mineral oil is an illustrative example of such an oil.

The proportions of oil and water employed in the microemulsion are not narrowly critical. The oil is used in an amount sufficient to dissolve enough tepoxalin so that the final concentration of the tepoxalin in the microemulsion will be sufficient to impart anti-inflammatory properties to the unit dosage quantities (e.g., from one to three drops of the microemulsion). This concentration of tepoxalin is usually within the range of from about 0.05 weight per cent to about 1.0 weight percent of the total weight of the aqueous emulsion of the invention. The upper limit of oil concentration is that concentration that will begin to impart irritation properties to the eyes of patients, or that concentration that will be too high to form a microemulsion. Generally, oil/water proportions of from about 1/99 to about 49/51 (v/v) will prove to be useful. In particular cases, optimum proportions of oil and water can be determined by routine experimentation.

The microemulsions of the invention also contain surfactants, additives to impart the proper tonicity to the microemulsion, buffers, preservatives, and other such additives and adjuvants that are known in the art. Examples of surfactants that can be used include sorbitan mono-oleate, NF (Span 80), sorbitan monostearate (Span 60), which are sorbitan fatty acid esters, polyoxyethylene 20 sorbitan monostearate (Tween 60), polyoxyethylene 20 sorbitan monooleate (Tween 80), which are polyoxyethylene sorbitan esters, Pluronic F127, which is a polyoxyethylene-polyoxypropylene block copolymer, sucrose stearate (Crodesta F-160), which is a sugar/fatty acid ester, and the like. The experimental section below gives illustrations of the types and proportions of surfactants and other additives that can be employed in the microemulsions of the invention.

The microemulsion employed in the invention can be prepared by first dissolving tepoxalin in the oil, mixing the oil/tepoxalin solution with a surfactant to form a homogeneous solution which is then mixed with water to form an oil/water mixture which is then subjected to appropriate energy such as sonication to form the microemulsion. If desired, additional tepoxalin can be added to the oil/water mixture after the microemulsion forming step. The following is a representative preparation procedure of a microemulsion of the invention wherein the energy applied to form the microemulsion is sonic energy:

General Procedure for Preparing a Tepoxalin Microemulsion

The oil such as castor oil is presaturated with tepoxalin (64.06:1 w/w, oil to drug) by stirring the mixture at 50°C until all the material is dissolved (about 16 hours).

The oil and surfactant (e.g., a polyoxyethylene sorbitan fatty acid ester such as Tween 80) are mixed together with a magnetic stir bar in a suitable container such as a plastic (e.g., polymethylpentene) beaker at ambient temperature until a homogenous solution is attained. The oil phase is then mixed with water and stirred until homogenous (cloudy emulsion).

The mixture is sonicated in a suitable container such as a 40 ml clear plastic beaker for 30 minutes with a sonicator such as a "VibraCell" sonicator set at maximum output with a 30% duty cycle (pulse rate); the pulser switch is set to the "on" position (turns the duty cycle on or initiates a discontinuous rate of sonication). The beaker is kept at a constant temperature via a water jacket controlled at a cool temperature such as about 5°C. A low temperature in the jacket is necessary in order to prevent the contents of the beaker from exceeding an optimum temperature needed for formation of the microemulsion (a higher or lower temperature in the beaker will prolong the time to form the microemulsion). The mixture is sonicated for abut 30 minutes or until the mixture becomes translucent, which is the indication that a microemulsion has formed. The temperature of the emulsion is not allowed to rise above a temperature within the range of from about 45°C to about 55°C. Additional tepoxalin (sufficient to bring the concentration of tepoxalin in the microemulsion up to about 0.1%, by weight) is added to the emulsion and the mixture is stirred until all the particles of the drug have dissolved. The emulsion is then filtered through a 0.2 $\mu$ membrane filter to remove any residual particles.

Disodium edetate (that is, the disodium salt of ethylenediaminetetraacetic acid) is slowly added to the emulsion while stirring until totally dissolved. Disodium edetate is a metal-chelator used to prevent the degradation of tepoxalin in the formulation. Aqueous BAK (Benzalkonium chloride - 50% w/v), NaCl, and buffer salts are then added to the microemulsion, in the following sequence:

The NaCl is added after BAK to adjust for tonicity in the range of 200 to 330 mOsm. The buffer is prepared in situ by adding and dissolving citric acid first and adjusting the final Ph with $Na_2HPO_4$ to 4.5 - 7.0. The final formulation is then filtered through a 0.2 micron membrane filter into sterile containers. The clarity of the microemulsion is determined by percent transmittance at 520 nm (H.L. Rosano, J.L. Cavallo and G.B. Lyons, Chapter 16, Microemulsion Systems, edited by H.L. Rosano and M. Clausse, Marcel Dekker, Inc., new York and Basel, Copyright 1987). The transmittance is preferably greater than 70%.

The Examples set forth below further illustrate the invention. In the Examples, the following materials and equipment were used:

Materials and Equipment:

Drugs, chemicals, and reagents used for the preparation of the microemulsion formulation of tepoxalin are listed below with their sources.

1.   Distilled water
2.   Tepoxalin                                          Ortho
3.   Castor oil                                         CasChem, Inc
     (Gold Bond Oil, conforms to USP XIX)
4.   Tween 60                                           Sigma Chemical
5.   Tween 80 (polysorbate 80 USP)                      Fisher
6.   Sodium Chloride Crystals AR                        Mallinckrodt
7.   Disodium Edetate USP                               Ciba Geigy
8.   Benzalkonium chloride 97% USP (BAK)                Henkel

9.   Citric acid USP, powder anhy.                      Pfizer
10.  Sodium Phosphate Dibasic USP (anhy.)               Mallinckrodt

The sonicator used in the Examples to form the microemulsions was a Sonics and Materials "VibraCell" model VC300. The sonicator probe is a 0.5 inch high-intensity threaded end type with a titanium tip. The microemulsion has to be kept at a constant temperature during sonication. The circulating system used is a MGW-/LAUDA type RSC 6 (Range -30° to 150°C) cooler/heater set to 5°C.

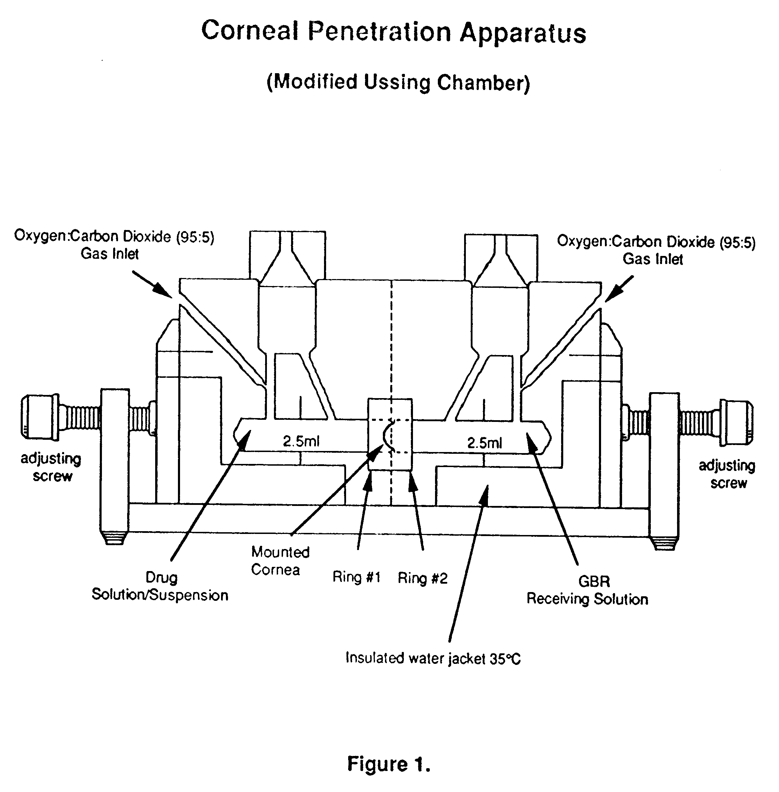

Modified Ussing type chambers (H.F. Edelhauser, J.R. Hoffert, P.O. Fromm, Invest. Ophthalmol. Vis. Sci., 4:290-296 (1965)) designed with 2.5 ml donor and 2.5 ml receiver cells (separated by the cornea which is mounted between two sealing rings) are used for the studies to determine penetration of the cornea by the microemulsions of the invention. The system is 2-chambered (in vitro cells) (see Figure 1) and is designed to monitor the penetration of drug entities across biological membranes. Usually the cells are temperature controlled by means of an exterior water jacket; the stirring of the dosing and receiving solutions in the cells is achieved by bubbling a gas through the cells. In the case of the cornea, the gas is a mixture of 5% $CO_2$ balanced with oxygen, to maintain physiological Ph.

Modified Glutathione Bicarbonate Ringer's solution (GBR) (R.D. Schoenwald and H.S. Huang, J.Pharm. Sci. 72(11) (1983) 1266-1271) is freshly prepared for the studies, by mixing equal volumes of two stock solutions which can be stored up to a week in a refrigerator prior to use. After reconstitution the solution should only be used for approximately 6 hours. During use, the pH of the mixture should be maintained at 7.4 by bubbling a 5% $CO_2/O_2$ mixture through it. The chemicals used for the preparation of GBR are listed below.

## Stock Solution I

| | | |
|---|---|---|
| NaCl | (14.2 g) | Fisher |
| KCl | (0.716 g) | Fisher |
| $NaH_2PO_4$ | (0.50 g) | Fisher |
| $NaHCO_3$ | (4.908 g) | Fisher |
| water | dilute to 1 liter. | |

## Stock Solution II

| | | |
|---|---|---|
| $CaCl_2 \cdot 2H_2O$ | (0.30 g) | Fisher |
| $MgCl_2 \cdot 6H_2O$ | (0.318 g) | Fisher |
| D(+)-glucose | (1.80 g) | Sigma |
| Reduced glutathione | (0.184 g) | Sigma |
| water - dilute to 1 liter. | | |

The corneas are obtained from adult albino rabbits of both sexes, weighing approximately 3 Kg.

The analysis of tepoxalin for the in vitro corneal penetration studies uses the following equipment:

1. Pump - Model 600E System Controller from Waters Associates;
2. Autosampler - 712 WISP from Waters Associates;
3. Integrator - SP4270 from Spectra Physics;
4. Detector - Perkin Elmer LS-5B Luminescence Spectrophotometer or a Waters 990 Photodiode Array Detector; and
5. Column - μBONDPAK C18 ( 30 cm L x 3. 9 mm I.D.) from Waters Associates.

The methodology to quantify tepoxalin in GBR was developed using reversed-phase high performance liquid chromatography (HPLC) and fluorescent detection. A GBR sample containing tepoxalin is quantified by adding the internal standard 4,5-diphenylimidazole to the sample, mixing and injecting an aliquot into the HPLC. A ratio of peaks heights of tepoxalin to internal standard is plotted against tepoxalin's standard concentrations to obtain a calibration curve.

The chemicals used are 2-propanol for the mobile phase and 4,5-diphenylimidazole 98% for the internal standard. The internal standard is a reference compound to tepoxalin used in the assay to compensate for any variations that might occur in the measurement of tepoxalin from sample to sample. The mobile phase partitions the analytes in and out of the stationary phase (in the analytical column).

Preparation of a tepoxalin microemulsion by sonication

The castor oil is presaturated with tepoxalin (64.06:1, w/w, oil to drug) by stirring the mixture at 50°C until all the tepoxalin is dissolved (about 16 hours).

The oil and surfactant (e.g., Tween 60) are mixed in a ratio of 1:2 v/v with a magnetic stir bar in a plastic (e.g., polymethylpentene) beaker at ambient temperature until a homogenous solution is attained. The oil phase is then mixed with water and stirred until homogenous (cloudy emulsion).

The mixture is sonicated in a 40 ml clear plastic beaker for 30 minutes with the "VibraCell" sonicator set at maximum output with a 30% duty cycle; the pulser is set to the "on" position. The beaker is kept at a constant temperature via a water jacket controlled at 5°C. The mixture is sonicated for 30 minutes or until the mixture becomes translucent ( a microemulsion ). The temperature of the emulsion is not allowed to rise above 52°C. Tepoxalin is added to the microemulsion and stirred until all the particles of the drug have dissolved. The emulsion is then filtered through a 0. 2 μ Tuffryn Membrane filter to remove any residual particles.

Disodium Edetate is slowly added to the emulsion while stirring (until totally dissolved). BAK (50% w/w), NaCl and buffer salts are then added, in this sequence. The NaCl is added after the BAK to adjust for tonicity in the range of 200 to 330 mOsm. The buffer is prepared in situ by adding and dissolving citric acid first and adjusting the final pH with $Na_2HPO_4$ to 4.4 - 7.0. The final formulation is then filtered through 0.2 micron Tuffryn Membrane into sterile containers. The clarity of the microemulsion is determined by percent transmittance at 520 nm (H.L. Rosano, J.L. Cavallo and G.B. Lyons, Chapter 16, Microemulsion Systems, edited by H.L. Rosano and M. Clausse, Marcel Dekker, Inc., New York and Basel, Copyright 1987). The transmittance should be greater than 70%.

Preparation of Microemulsion Using Tween 60 as Surfactant

Procedure A -- (flexible procedure that allows for changes in pH, tonicity, etc.) -- Castor oil is presaturated with tepoxalin by mixing 100 ml of the oil with 1.5 g of tepoxalin for 16 hours at 50°C. An aliquot of 0.3 ml of the oil and 0.6 ml of Tween 60 are mixed together to a homogenous solution (as described above) and then are mixed with 28.82 ml of water giving a cloudy emulsion.

The emulsion is then sonicated as previously described to a translucent solution. The microemulsion is stirred with 0.03 g of tepoxalin for about 1 hour until there are no particles remaining. The solution is filtered, and 0.009 g of disodium Edetate, 3-6 µl of BAK (50%), 0.20 g of NaCl, 0.001 g of citric acid and 0.005 g of Na2HPO4 are added/titrated into the microemulsion to give the tonicity and pH described previously.

Procedure B -- (Less flexible procedure for making adjustments, but is easier to use for the preparation of the microemulsion) -- 0.3 ml of castor oil is mixed with 0.6 ml of Tween 60 and stirred at ambient temperature until homogenous. The temperature of the oil mixture is raised to 50°C and 0.03 g of tepoxalin is added while stirring. The oil continues to be stirred at 50°C until all the tepoxalin is completely dissolved (approximately 1 hour).

The aqueous phase that will be mixed with the oil mixture in order to form the emulsion is made by dissolving 0.2 g NaCl, 0.009 g of disodium Edetate, 0.001 g citric acid, 0.005 g dibasic sodium phosphate, and 12 µl of BAK (50%) in 25 ml water. The solution is stirred until all of the excipients dissolve. The oil mixture is then added to the aqueous phase and mixed well until a cloudy emulsion is formed. The solution is diluted further with 5 ml of water to 30 ml while stirring.

The crude emulsion is sonicated to a microemulsion as described in Procedure A.

The following table displays a summary of solubility studies carried out on tepoxalin in various vehicles:

Table __1__ : Solubility of Tepoxalin in Various Oils, Surfactants, HP-ß-Cyclodextrin, and Combinations Thereof.

| Matrix | | Aqueous Concentration (Percent) | Tepoxalin Solubility (Percent) |
|---|---|---|---|
| Water | | Neat | 0.00044 |
| Oils | sesame | Neat | 0.4 |
| | castor | Neat | 1.6 |
| | mineral | Neat | 0.0 |
| | sunflower Seed | Neat | 0.4 |
| | Cottonseed | Neat | 0.06 |
| | perfluoro-decalin | Neat | 0.0 |
| Surfactants | Pluronic P-105 | 6% w/w | 0.099 |
| | Pluronic F-127 | 6% | 0.035 |
| | Tween 80 | 6% | 0.120 |
| | Tween 80 | 3% | 0.099 |
| | Tween 80 | 2% | 0.089 |
| | Tween 80 | 1% | 0.030 |
| | Tween 40 | 2% | 0.066 |
| | Tween 20 | 2% | 0.053 |
| | Tween 60 | 2% | 0.079 |
| Surfactant and HP-ß-CD Solution | HP-ß-CD, Tween 80 | 5% 2% | 0.097 |
| | HP-ß-CD, Tween 80 | 10% 1% | 0.113 |
| Oil, Surfactant Microemulsion | castor oil, Pluronic P-105 | 3% 7% | 0.108 |
| | castor oil, Tween 80 (tepoxalin is added after microemulsion) | 1% 2% | 0.085 |
| | castor oil, Tween 80 | 2% 2% | 0.106 |
| | castor oil, Tween 80 (tepoxalin is added before emulsion is prepared) | 1% 2% | 0.147 |
| | castor oil, Tween 60 | 1% 2% | 0.15 |

## Preparation of a Tepoxalin Microemulsion using Tween 80 as the Surfactant and Span 80 as the Cosurfactant

The procedure to prepare a microemulsion with Tween 80/Span 80 is identical to the Tween 60 emulsion except Span 80 is added just after formation of the translucent solution with Tween 80. An oil/water (o/w) system is titrated with the surfactant and cosurfactant to a hydrophilic-lipophilic balance (HLB) to obtain a "one-phase transparent o/w dispersion" (H.L. Rosano, J.L. Cavallo, and G.B. Lyons, Chapter 16, Microemulsion Systems, vol. 24, editor H.L. Rosano and M. Clause, Marcel Dekker, Inc., New York and Basel, 1987, page 271).

An aliquot of 0.3 ml of castor oil (containing tepoxalin) and 0.55 ml of Tween 80 are premixed to a homogenous solution. The solution is prepared as described previously to a microemulsion containing tepoxalin. An aliquot of 0.05 ml of Span 80 is then added to the microemulsion, and the mixture is sonicated further for 10 minutes. The microemulsion is treated with disodium Edetate, BAK, NaCl, and buffer salts as described above.

## In vitro Corneal Transport of Tepoxalin Via a 0.1% Suspension or a 0.1% Microemulsion

The eyes were enucleated with the conjunctival sac and lids attached. The cornea was excised and mounted using the Dikstein and Maurice Technique (S. Dikstein and D.M. Maurice, J. Physiol., 221: 29-41 (1972)).

The chambers were prepared by equilbrating them at 35°C, by means of a water jacket. Both the receiver and donor sides of the chamber were aerated and mixed with an oxygen:carbon dioxide (95:5) mixture; this maintains the pH of the modified glutathione bicarbonate Ringer's solution (GBR) at pH 7.4 and provides mixing of the volumes. The mounted corneas were assembled in the chamber; the receiver side filled with 35°C aerated GBR and the donor side filled with the test compound in GBR.

100 µl Aliquots were sampled from the receiver side over a 4-hour period (0, 15, 30, 45, 60, 90, 120, 180 and 240 minutes). The volume of the chamber was maintained by replenishing with GBR (kept at 35°C during the study), after each sample. The samples were immediately frozen in dry ice, so as to prevent any further metabolism occurring. At the end of the study, the bulk donor and receiver volumes were collected and also frozen. The mounted corneas were removed from the cells and the percentage hydration calculated by weighing the isolated tissue before and after drying (the corneas were placed in an oven overnight at 45°C). This hydration value is indicative of the condition of tissue (preferable range 75 to 83%). Untreated corneas were assessed as controls.

The samples were maintained at -70°C until analysis by HPLC.

## Bioanalytical Assay for Tepoxalin in GBR

The assay involves reversed-phase high performance liquid chromatography (HPLC) using fluorescent detection. The pump is set at a flow rate of 1.0 ml/min., the autosampler is set with an injection volume of 10 µl, the integrator is set with an attenuation of 8 millivolts (full-scale) and the fluorometer is set with a fix scale of 30, an excitation wavelength of 282 nm and an emission wavelength at 418 nm. The C18 column is kept at ambient temperature during analysis. The mobile phase is prepared by mixing 400 ml of isopropanol and 2 ml of $H_3PO_4$ with water to 900 ml. The pH is then adjusted to 3.0 with 1 M NaOH, and the final volume is a dilution with water to 1 liter. The mobile phase is degassed by filtration through a 0.45 µm Nylon-66 filter (from Rainin Instruments Co., Inc.) under vacuum.

Standards for the calibration curve are prepared in balanced salt solution (BSS) at 0.030, 0.060, 0.150, 030 and 0.60 µg/ml. The standards and samples are prepared for HPLC analysis by transferring to autosampler vials (limited volume inserts) 100 µl of the sample and 100 µl of the internal standard 4,5-diphenylimidazole (at 9 µg/ml). The vials are capped and mixed by a Vortex mixer (American Scientific Products). The mixer spins the contents of a vial rapidly or causes a vortex (circular motion).

The chromatography involves µBONDPAK C18 column with 10 µm particles and the mobile phase consists of 40% isopropanol (IPA) and a phosphate buffer adjusted to pH 3.0. A C18 column with a heavier carbon-load or a mobile phase containing a more polar organic solvent (e.g. methanol) will not elute the drug off the C18 column. The drug can be detected by ultra-violet absorbance (254 nm) or by fluorescence (excitation wavelength at 282 nm and an emission at 518 nm). Fluorescent detection is about 10 times more sensitive than ultra-violet.

A GBR sample is prepared for HPLC analysis by transferring an aliquot to an autosampler vial, adding the internal standard 4,5-diphenylimidazole, capping and mixing the vial. A flow rate of 1.0 ml/min will elute the internal standard in about 4.0 minutes and the drug in about 11.6 minutes. A calibration curve with standards between 0.030 and 0.60 µg/ml will give a linear curve using fluorescence detection with a correlation coefficient of r=0.9998 ($r^2$=0.9997), a Y-intercept = -0.0059 and a slope = 1.240. Minimum sensitivity is about 0.025 µg/ml.

Solubility Experiments with Tepoxalin

The low aqueous solubility of tepoxalin prompted solubility experiments with oils, surfactants, hydroxypropyl-beta-cyclodextrin, and combinations thereof. These experiments are summarized above in Table 1. A tepoxalin content of greater than 0.1% could be attained with a microemulsion using 1% castor oil and 2% of a Tween (polysorbate). An oil:surfactant ratio of 1:1 does not form a translucent emulsion (cloudy). The sequence of adding, mixing, and sonicating the drug and excipients is important in attaining a concentration greater than 0.1%.

Two representative microemulsions containing 0.1% tepoxalin have been prepared and tested for physical stability, drug stability, acute toxicity, and/or efficacy. The composition of the two emulsions are listed below:

## With Tween 60

| | | |
|---|---|---|
| tepoxalin | 0.030 g | 0.1% |
| castor oil | 0.300 ml | 1.0% |
| Tween 60 | 0.600 ml | 2.0% |
| NaCl | 0.200 g | 0.67% |
| disodium Edetate | 0.009 g | 0.03% |
| BAK (50% w/v) | 0.003 ml | 0.01% |
| citric acid | 0.001 g | 0.003% |
| $Na_2HPO_4$ | 0.005 g | 0.0167% |

The above is diluted to 30 ml with purified water.

## With Tween 80/Span 80

| | | |
|---|---|---|
| tepoxalin | 0.03 g | 0.1% |
| castor oil | 0.300 ml | 1.0% |
| Tween 80 | 0.55 ml | 1.833% |
| Span 80 | 0.05 ml | 0.166% |

The remaining excipients and diluent are as listed above.

Acute Multiple-Dose Toxicity Studies in Rabbits

(Ocular Irritancy Testing)

The acute irritancy of the Tween 80/Span 80 and the Tween 60 microemulsions were evaluated by subjecting the rabbits to multiple doses (1 drop/20 minutes for 6 hours/day) of the two formulations and other appropriate controls. The irritancy of the formulation was determined by the number of rabbits (3 rabbits/formulation) that passed a grading system (e.g. redness, swelling, tearing, etc.) for one day, two days, or three days. The degree of irritancy was scored between 1 and 10: a score of 1 was least irritating and a score of 10 was the most irritating.

Neat castor oil gave a score of 10. All other formulations and the control 10% Pluronic F127 (poloxamer 407, excipient in Vasocidin® at 0.1%) gave a score of 1 (see Table 2, below).

TABLE ___2___ : Summary of Multidose Irritancy Studies in Rabbits. There are three rabbits/formulation that can pass the grading system for the first day, the second day, and the third day.

| | Day (1) | Day (2) | Day (3) | Score |
|---|---|---|---|---|
| castor oil, USP | 0 | 0 | 0 | 10 |
| 10% Tween 80 | 3 | 3 | 3 | 1 |
| 10% Pluronic F127 | 3 | 3 | 3 | 1 |
| 0.1% tepoxalin (Tween 80 Micro-emulsion) | 3 | 3 | 3 | 1 |
| 0.1% tepoxalin (Tween 80 Micro-emulsion) | 2 | 2 | 2 | (?)*1 |
| Microemulsion w/o drug, (Tween 80) (no BAK) | 3 | 3 | 3 | 1 |

(*Animal removed from test; broke its back in retainer)

================================================================

| | Day (1) | Day (2) | Day (3) | Score |
|---|---|---|---|---|
| 0.1% Microemulsion (Tween 80/Span 80) | 3 | 3 | 3 | 1 |
| Tween 80/Span 80 Microemulsion (w/o drug) | 3 | 3 | 3 | 1 |
| 0.1% Microemulsion (Tween 80) | 3 | 3 | 3 | 1 |
| 10% Solution of Pluronic F127 (No BAK in formulations) | 3 | 3 | 3 | 1 |

================================================================

| | Day (1) | Day (2) | Day (3) | Score |
|---|---|---|---|---|
| 10% Pluronic F127 | 3 | 3 | 3 | 1 |
| 0.1% tepoxalin $\mu$ emulsion (Tween 60, BAK & EDTA) | 3 | 3 | 3 | 1 |
| Placebo $\mu$ emulsion (Tween 60, BAK & EDTA) | 3 | 3 | 3 | 1 |
| 0.1% tepoxalin $\mu$ emulsion (Tween 80, Span 80, BAK & EDTA) | 3 | 3 | 3 | 1 |
| 0.1% tepoxalin $\mu$ emulsion (Tween 80, Span 80) | 3 | 3 | 3 | 1 |
| 0.1% tepoxalin $\mu$ emulsion (Tween 60) | 3 | 3 | 3 | 1 |

In Vitro Cytotoxicity Testing of Tepoxalin Microemulsions

Four microemulsion formulations containing tepoxalin and three controls were tested for cytotoxicity to 3T3-L1 cells (embryo, mouse). A dose-response curve was constructed to determine the ID20, ID50 and ID80 of each formulation that "reduced the final total cellular protein by 20, 50 and 80% in comparison with that of the control wells" (from a report issued to D.G. Musson on March 23, 1990 from Jane Greeves, Sandra Reading and Clive Wilson, Department of Physiology and Pharmacology, Medical School, Queen's Medical Centre, Nottingham, NG7 2UH). The data showed (a) enhanced toxicity with formulations containing BAK, (b) all the formulations were more toxic than the control Pluronic F127, (c) the microemulsion with Tween 80/Span 80 appears slightly less toxic than Tween 60, and (d) emulsions with tepoxalin are more toxic than those without.

Stability Studies of Tepoxalin and the Microemulsion:

Two stability studies have been performed with the second still on-going. The first study involved three formulations:
(1) 0.1% tepoxalin/Tween 60/castor oil/BAK/NaCl
(2) 0.1% tepoxalin/Tween 60/Span 60/castor oil/BAK/NaCl
(3) 0.1% tepoxalin/Tween 80/Span 80/castor oil/BAK/NaCl
The study demonstrated several problems with the formulations and differences between the formulations. Emulsion stability and drug stability seemed to favor the Tween 60 surfactant without a co-surfactant. The rate of degradation of tepoxalin at 45°C seemed to be much greater than at room temperature and 35°C. Similarly, the cracking of the emulsion seemed to occur much faster at 45°C over room temperature and 35°C.

The results of the first stability study prompted an investigation for a way to reformulate. Tepoxalin appears to hydrolyze at the amide group to form a carboxylic acid. An accelerated procedure has been developed to observe this degradation within 16 hours. The sample is analyzed by reversed-phase HPLC using a diode array for detection. A series of experiments (following section) suggested the use of 0.03% disodium Edetate in the formulation. With disodium Edetate in the microemulsion, the drug degraded at and above pH 7.0; degradation was minimal at pH's 5.0, 5.5 and 6.0. Thus, a second stability study was begun with 0.03% disodium Edetate in the formulation and the pH adjusted to 5.9 - 6.1 with a citric acid/sodium phosphate dibasic buffer.
The second stability study employed two formulations:
(4) 0.1% tepoxalin/Tween 60/castor oil/BAK/NaCl/disodium Edetate/citric acid/$Na_2HPO_4$/water
(5) 0.1% tepoxalin/Tween 80/Span 80/castor oil/BAK/NaCl/disodium Edetate/citric acid/$Na_2HPO_4$/water.
There is three months of data. The drug stability data is acceptable at room temperature and 35°C for formulation (4). The latter formulation (5) with Tween80/Span80 resulted in tepoxalin concentrations below the acceptable 90% level at 12 weeks at 35°C; the Tepoxalin concentration at 12 weeks at room temperature was 94.4%. The pH's for both formulations have dropped from 6.0 to 4.85 - 5.76, depending on the temperature. The percent transmittance appear to have dropped slightly at room temperature and 35°C for both formulations; at 45°C, the drop has been significant.

Experiments to Enhance Stability of Tepoxalin Microemulsion

A number of experiments were performed in succession in order to elucidate a mechanism for the degradation of tepoxalin in water and in the microemulsion and to determine optimum conditions for the tepoxalin microemulsion. The drug is suspected of vulnerability to acid and base catalysis of its N,N-hydroxymethyl amide group forming a carboxylic acid. The experiments and results:
(a) 0.1% tepoxalin was dissolved in 50:50 water: ethanol and aliquots were pH adjusted with 1M NaOH to 5.0, 6.0, 7.0, 8.0 and 12.6. The aliquots were heated in capped reaction vials for 16 hours at 100°C.
The HPLC chromatograms show a degradation peak eluting after tepoxalin in samples at pH 6, 7, 8, 12.6 but not at pH 5.0.
(b) 0.1% tepoxalin was prepared with castor oil, Tween 60 and a citrate/phosphate buffer (1 M) in the form of a microemulsion. Two drops of 1M citric acid solution were added to an aliquot (1 ml) and the pH was adjusted with 1.0 M sodium phosphate dibasic to pH 5.0, 6.0, 7.0, and 7.6. The samples were heated as before.
The chromatograms show two major degradation products: one eluting before tepoxalin and another after. The degradation occurs greatest at pH 5.0 and 7.6.
(c) The previous experiment (b) was repeated narrowing the pH range between 6.0 and 7.0 (6.0, 6.2, 6.4, 6.6, 6.8, and 7.0).
The optimum pH appears between pH 6.2 and 6.4, but there is still significant degradation.

(d) The previous experiment (c) was repeated with disodium Edetate 0.03% in each sample and with the same pH range.

Degradation is minimal at pH 6.0 and increases with pH to 7.0. The presence of the second degradate that elutes before tepoxalin is also minimal.

(e) Experiment (d) was repeated with a broader pH range: 5.0, 5.5, 6.0, and 7.0.

Degradation was minimal at pH 5.0, 5.5, and 6.0. Peak areas and heights for tepoxalin at these pH's were:

| pH | Peak Areas | Peak Heights |
|-----|------------|--------------|
| 5.0 | 0.50144 | 0.7807 |
| 5.5 | 0.55158 | 0.8312 |
| 6.0 | 0.56796 | 0.8390 |
| 7.0 | 0.31217 | 0.5556 |

and reflect optimum stability between 5.5 and 6.0. The degradation product was not clearly evident except at pH 7. 0.

## In Vitro Corneal Penetration Studies:

The penetration and metabolism of tepoxalin across the rabbit cornea as a suspension at 0.1% and as a microemulsion at 0.1% were compared by an in vitro procedure using the modified Ussing Chambers (H.F. Edelhauser, J.R. Hoffert, P.O. Fromm, Invest. Ophthalmol. Vis. Sci., 4 (1965) 290-296). The samples were removed at appropriate time intervals from 0 up to 240 minutes and frozen at -70°C until analysis by HPLC-fluorescence.

A plot of the amount of tepoxalin crossing the cornea against time showed the drug penetrating the cornea from both formulations. The chromatograms of the samples taken from the receiver cells also show other metabolites/-degradates present via both formulations. The metabolites/-degradates could be chemical and enzymatic hydrolysis of the N,N-hydroxymethylamide group to a carboxylic acid. The tepoxalin fluxes for both formulations are listed below:

| | Flux $(\mu g/cm^2\text{-min})$ |
|---|---|
| Suspension | 0.00384 |
| Microemulsion | 0.00959 |

The flux was calculated using the following equation (C. Fleeker, O. Wong, and J.A. Rytting) Pharm. Res., 6 (6) (1989) 443-448:

Flux $= (\Delta q/\Delta t)(1/A)$, where

$(\Delta q/\Delta t)$ = slope, and

A = corneal surface exposed to the drug, 1.039 $cm^2$.

For the Suspension, the slope was determined between 45 and 180 minutes; for the microemulsion, between 60 and 240 minutes.

## Efficacy Studies in Cats:

Eight cats with heavily pigmented irides are pretreated with the 0.1% tepoxalin microemulsion containing the surfactants Tween 80/Span 80 in the right eye and control saline in the left eye. The cats are anesthetized fifteen minutes before completion of the dosage regimen and both irides are subjected to an argon laser. The effects of the drug and control solutions on the irides are observed by slit-lamp. The animal model is most informative for drug effect between 4 and 8 hours.

For iris hyperemia, there was a significant statistical effect in the treated eyes over controls at 4, 6, and 8 hours, there was no difference in the treated eyes and control eyes at 24 hours.

For iris edema, there was a significant statistical effect in the treated eyes at 6 hours.

An ophthalmic microemulsion formulation with tepoxalin would contain (concentrations are w/w):

(1) an oil immiscible with water at 0.1 to 2%;

(2) a non-ionic surfactant at 0.1 to 4%;

(3) a non-ionic co-surfactant at 0.0 to 4%;

(4) tepoxalin at 0.05 to 1%;

(5) tonicity agent at 0.25 to 2%;

(6) a buffering system to adjust the pH between 4.0 and 7.0 (above pH 7.0, the drug degrades). The concentration should vary from 0 to 3.0 millimolar;

(7) one or more preservatives at 0.02 to 0.7%; and

(8) a stabilizer for the microemulsion and/or the drug at 0.0 to 1.0%.

The above materials are mixed in such a manner by sonication, by homogenization, or by a technique using a microfluidizer, to obtain a translucent to transparent microemulsion (transmittance at 520 nm is >=70%).

Two illustrative formulations are the following:

## Formulation A

| | | |
|---|---|---|
| 1. | tepoxalin | 0.1% |
| 2. | castor oil | 1.0 |
| 3. | Tween 60 | 2.0 |
| 4. | NaCl | 0.67 |
| 5. | disodium edetate | 0.03 |
| 6. | BAK | 0.03 |
| 7. | citric acid | 0.003 |
| 8. | $Na_2HPO_4$ 1.18 millimolar, | 0.0167 |
| 9. | water | diluted to 1 liter; |

## Formulation B

| | | |
|---|---|---|
| 1. | tepoxalin | 0.1% |
| 2. | castor oil | 1.0 |
| 3. | Tween 80 | 1.83 |
| 4. | Span 80 | 0.166 |

The remaining excipients and diluent are as listed above for Formulation A.

One or both formulations have been tested for drug stability, emulsion stability, toxicity ( irritation ), in vitro corneal penetration, and efficacy.

The materials used for the above two illustrative emulsions can be replaced with the following substitute materials, which are listed for illustrative purposes:

OILS

sesame oil
castor oil
mineral oil
sunflower seed oil
perfluoro-decalin
almond oil NF
apricot kernel oil
avocado oil
coconut oil
cross-essential EPO
menhaden oil
mink oil

olive oil
orange roughy oil
safflower oil USP
wheat germ oil

SURFACTANTS and CO-SURFACTANTS

polysorbates (polyoxyethylene sorbitan fatty acid esters:
Tweens 20, 21, 40, 60, 61, 65, 80, 81, 85);
sorbitan esters (sorbitan fatty acid esters: Span 20, 40, 60, 65, 80, 85);
Pluronics (P84, P105, F127);
Tetronics;
Crodestras (Combination of sucrose stearate and sucrose distearate, sucrose stearate);
lecithin,

TONICITY AGENTS

sodium chloride,
potassium chloride,
dextrose, glycerin, mannitol

BUFFERS

acetic acid,
boric acid,
hydrochloric acid,
citric acid,
phosphoric acid,
potassium carbonate,
potassium citrate,
potassium phosphates,
sodium acetate,
sodium bicarbonate,
sodium biphosphate,
sodium borate,
sodium carbonate,
sodium citrate,
sodium hydroxide,
sodium phosphate.

PRESERVATIVES

benzalkonium chloride,
benzethonium chloride,
chlorobutanol,
phenylmercuric acetate,
phenylmercuric nitrate,
thimerosal,
methylparaben,
propylparaben,
sodium benzoate,
sorbic acid,
phenylethyl alcohol,
boric acid,
gentamicin sulfate,
Bacitracin,
polymixin B sulfate,
Neomycin,

tetracycline hydrochloride,
erythromycin,
sulfacetamide sodium,
tobramycin,
Prednisolone acetate,
Prednisolone,
Prednisolone phosphate,
dexamethasone,
and combinations of the above.

STABILIZERS

disodium edetate (metal-chelating agent),
citric acid (reducing agent),
sodium metabisulfite (reducing agent),
ascorbic acid (reducing agent),
acetyl cysteine (reducing agent),
butylated hydroxyanisole (radical scavenger),
2,6-di-tert-butyl-p-cresol (radical scavenger),
vitamin E (radical scavenger).

Solubility experiments with formulations A and B suggest that much of the drug is located in the interface between the oil droplet, surfactant(s), and water. Maximum tepoxalin solubility for castor oil is 1.6%, and 1% of the oil is dispersed in the emulsion. Thus, 0.134% of the 0.15% drug in the emulsion resides in the interface.

The first stability study involved three formulations that contained different surfactants, were not buffered, and had no stabilizers. At 45°C versus room temperature and 35°C, both the concentrations of tepoxalin and the transmittances of the emulsions declined dramatically. All of the formulations tested for accelerated tepoxalin stability failed within two weeks at 45°C (below 90% of initial concentrations) and all failed within 8 weeks at 35°C. A method, therefore, was developed to investigate the degradation of tepoxalin in order to reformulate a stable product.

Initial stability experiments seemed to indicate that the emulsion system is accelerating the degradation of tepoxalin, forming two major by-products across the pH range of 5 to 7.6; minimum degradation occurs at 6.2 - 6.4. Degradation of tepoxalin in water/alcohol gave one major by-product at pH 6.0 or higher. Edetate in the emulsion stabilized tepoxalin in the pH range of 5.0 - 6.0 and only one major by-product was observed.

A second stability study is being conducted with two formulations, both containing edetate and a buffer, pH 5.9 - 6.1. As before, tepoxalin is degrading significantly faster at 45°C than at room temperature and 35°C. After three months of data collection, the percent remaining of tepoxalin is significantly higher compared to the previous study. The concentration change for tepoxalin at 35°C is acceptable at 90.3% using Tween 60 and at room temperature, at 98.3%; percent remaining at 45°C is 70.7%. Shelf-life for the two formulations will be determined at temperatures lower than 45°C.

The stability of tepoxalin and the physical stability of the emulsion appear to be related and dependent on temperature. The data from both stability studies and the experimental investigations indicate that at higher temperatures the emulsions are cracking faster and the drug is degrading. The formulations with Tween 60 as compared to formulations with Tween 80 in both studies are more stable in both aspects. In addition, reformulation with edetate and buffer does stabilize both the drug and the emulsion.

The acute multiple-dose toxicity tests basically indicate that the formulations containing Tween 60 or Tween 80/Span 80 are non-irritating. The in vitro cytotoxicity tests are more discriminating, showing that the microemulsions with tepoxalin and/or BAK are more cyto-toxic (cellular death) than without. The difference between the formulations containing Tween 60 and Tween 80/Span 80 is not significant considering the standard deviations.

The efficacy studies basically show that the 0.1% tepoxalin emulsion is effective for the reduction in iris swelling at 6 hours and in hyperemia at 4, 6, and 8 hours in eyes traumatized by an argon laser.

## Claims

1. An ophthalmic composition suitable for topical application to the eye comprising an oil in water microemulsion containing tepoxalin in an anti-inflammatory effective concentration.

2. The ophthalmic composition of Claim 1 wherein the oil is a fixed oil.

3. The ophthalmic composition of Claim 1 wherein the microemulsion additionally contains a non-ionic surfactant in an amount effective to stabilize the microemulsion.

4. The ophthalmic composition of Claim 1 wherein the microemulsion additionally contains tonicity agents, buffers, and stabilizers.

5. The ophthalmic composition of Claim 2 wherein the oil is castor oil.

6. The ophthalmic composition of Claim 3 wherein the non-ionic surfactant is a polysorbate.

7. The ophthalmic composition of Claim 4 wherein the microemulsion contains sodium chloride in an amount sufficient to adjust tonicity between 200 and 330 mOsm.

8. The ophthalmic composition of Claim 4 wherein the microemulsion contains disodium edetate in an amount sufficient to enhance the stability of the tepoxalin contained in said composition.

9. The ophthalmic composition of Claim 5 wherein the microemulsion contains a phosphate buffer to adjust the pH between about 4.0 and 7.0.

**Claims for the following Contracting State : ES**

1. A method for preparing an ophthalmic composition suitable for topical application to the eye, which method comprises the step of forming an oil in water microemulsion containing tepoxalin in an anti-inflammatory effective concentration.

2. A method according to claim 1 wherein the oil is a fixed oil.

3. A method according to claim 1 or 2 wherein the microemulsion additionally contains a non-ionic surfactant in an amount effective to stabilize the microemulsion.

4. A method according to claim 1, 2 or 3 wherein the microemulsion additionally contains tonicity agents, buffers, and stabilizers.

5. A method according to any preceding claim wherein the oil is castor oil.

6. A method according to claim 3 wherein the non-ionic surfactant is a polysorbate.

7. A method according to any preceding claim wherein the microemulsion contains sodium chloride in an amount sufficient to adjust tonicity between 200 and 330 mOsm.

8. A method according to any preceding claim wherein the microemulsion contains disodium edetate in an amount sufficient to enhance the stability of the tepoxalin contained in said composition.

9. A method according to any preceding claim wherein the microemulsion contains a phosphate buffer to adjust the pH between about 4.0 and 7.0.

# Corneal Penetration Apparatus

## (Modified Ussing Chamber)

Figure 1.

**European Patent Office**

# EUROPEAN SEARCH REPORT

Application Number

EP 91 30 9243

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 248 594 (ORTHO PHARM. CORP.) <br> * Claims 1,10-11; page 6, lines 55-57; page 7, lines 20-25,33-37,50-52 * <br> --- | 1-4 | A 61 K 9/107 <br> A 61 K 9/06 <br> A 61 K 31/415 |
| D,A | EP-A-0 253 472 (GREEN CROSS) <br> * Claims 1,3-8,11,15; page 4, lines 40-56; page 5, lines 1-14 * <br> ----- | 1-4 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 25-11-1991 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)